# EUROPÄISCHE PATENTSCHRIFT

(11) **EP 2 024 255 B1**
(45) Veröffentlichungstag und Bekanntmachung des Hinweises auf die Patenterteilung: **29.06.2011**
(21) Anmeldenummer: 07724713.8
(22) Anmeldetag: 28.04.2007
(51) Int. Cl.: B65D 83/08, B65D 77/02, B65D 75/58, A61F 15/00

(54) **BEUTEL-SAMMELPACKUNG**
POUCH-BASED CUMULATIVE PACKAGING
EMBALLAGE COLLECTIF DE SACHETS

(30) Priorität: 12.05.2006 DE 102006022198
(43) Veröffentlichungstag der Anmeldung: 18.02.2009
(73) Patentinhaber: LTS LOHMANN Therapie-Systeme AG, 56626 Andernach (DE)
(72) Erfinder: HACKBARTH, Ronald, 56075 Koblenz (DE)
(74) Vertreter: Schmidt, Werner
(86) Internationale Anmeldenummer: PCT/EP2007/003786
(87) Internationale Veröffentlichungsnummer: WO 2007/131615

(56) Entgegenhaltungen:
- EP-A- 0 948 949
- EP-A2- 0 101 298
- DE-U1- 9 418 312
- US-A- 4 194 624
- US-B1- 6 708 826

## Beschreibung

Die Erfindung betrifft eine Verpackungseinheit, die mindestens ein wirkstoffhaltiges Produkt, mindestens eine Primärverpackung und eine Sekundärverpackung umfasst, wobei zumindest ein wirkstoffhaltiges Produkt in jeder geschlossenen Primärverpackung versiegelt gelagert ist und wobei die Primärverpackungen in der Sekundärverpackung gestapelt angeordnet und mittels Solltrennfugen zumindest bereichsweise lösbar befestigt sind, wobei jede Primärverpackung ein Siegelbeutel mit einem Griffstück ist, der einzelne Siegelbeutel eine Solltrennnaht hat, die einen Aufreißbereich begrenzt und das Griffstück mit dem Aufreißbereich verbunden ist.

Aus der EP 1 161 375 B1 ist eine derartige Verpackungseinheit bekannt. Die einzelnen Primärverpackungen werden in einer vorgegebenen Reihenfolge aus der Sekundärverpackung entnommen. Nach der Entnahme werden die einzelnen Primärverpackungen geöffnet und das jeweilige wirkstoffhaltige Produkt entnommen. Hierbei können die wirkstoffhaltigen Produkte beschädigt werden. Außerdem können die Primärverpackungen ungeöffnet auf Vorrat entnommen werden und beispielsweise von Kindern unbedacht geöffnet werden.

Aus der EP 0 101 298 ist ein verpacktes, selbsthaftendes Heftpflaster bekannt, wobei das genannte Pflaster eine biegsame, ebene Unterlage aufweist, die auf einer ihrer Oberflächen mit einem Haftkleber beschichtet ist, sowie ein Kissen zum Absorbieren von Körperexsudat, welches Kissen auf der mit dem Haftkleber beschichteten Oberfläche der Unterlage angeordnet ist. Das genannte Pflaster ist unter Bildung eines gefalteten Pflasters, welches auf jeder seiner Hauptaußenseiten mit einem Haftkleberüberzug versehen ist, um eine Querfaltungslinie auf sich selbst gefaltet und wobei das gefaltete Pflaster unter Bildung eines mit einer Abdeckung versehenen, gefalteten Pflasters zwischen Abdeckblätter eingelegt ist, welche Abdeckblätter an dem genannten Haftkleber anhaften. Weiterhin ist das Pflaster von einer Umhüllung umschlossen, wobei Teile der Innenseiten der Umhüllung über den Abdeckblättern zu liegen kommen. Das Pflaster weist weiterhin Versiegelungsmittel auf, mittels welcher die genannten Teile der Innenseite der Umhüllung an den Abdeckblättern anhaften und wobei die Versiegelungsmittel eine größere Klebrigkeit zwischen den genannten Teilen und den Abdeckblättern schaffen als die Klebrigkeit des Klebers zwischen den Abdeckblättern und dem gefalteten Pflaster ausmacht. Wenn die Verpackung durch entfernen der Umhüllung geöffnet wird, werden sich die Abdeckblätter von dem gefalteten Pflaster ablösen und an der Umhüllung anhaften bleiben, wodurch sich das Pflaster gebrauchsfertig darbietet.

Die US 6,708,826 B1 zeigt eine Verpackungseinheit, bei der der Aufnahmebereich für das wirkstoffhaltige Produkt in der Primärverpackung eine Aufreißnaht aufweist, die identisch ist mit der Befestigungsnaht der Primärverpackung in der Sekundärverpackung. Beim Abreißen der Primärverpackung wird gleichzeitig das Produktnest aufgerissen, wobei ein Stück dieses Produktnestes abgerissen wird. Kann das wirkstoffhaltige Produkt nicht ohne Weiteres entnommen werden, kann es nach zusätzlichen Abreiß- oder Aufreißvorgängen aus der Primärverpackung herausgepult werden. Da bei all diesen Reißvorgängen das Produktnest angerissen wird, kann das wirkstoffhaltige Produkt beschädigt werden oder herausfallen.

Der vorliegenden Erfindung liegt daher die Problemstellung zugrunde, eine Verpackungseinheit zu entwickeln, bei der die wirkstoffhaltigen Produkte kindersicher verpackt sind und weitgehend ohne Gefahr einer Beschädigung problemlos entnommen werden können.

Diese Problemstellung wird erfindungsgemäß durch eine Verpackungseinheit gemäß dem Oberbegriff von Anspruch 1, wobei der untere und obere Packstoff an der der Solltrennfuge abgewandten Seite des Beutels mittels einer querliegenden Falte unlösbar miteinander verbunden sind, wobei die Solltrennnähte des Siegelbeutels eine auftrennbare gesiegelte Quernaht an der der Solltrennfuge zugewandten Seite des Beutels und zwei in Zugrichtung des Griffstücks orientierte auftrennbar gesiegelte Längsnähte sind, die das Produktnest begrenzen, und wobei das Griffstück auf dem oberen Packstoff aufliegt und mit diesem an der der Solltrennfuge zugewandten Seite des Beutels mittels einer querliegenden Falte unlösbar verbunden ist, oder durch eine Verpackungseinheit gemäß dem Oberbegriff von Anspruch 2, wobei der obere Packstoff entlang einer Linie mechanisch geschwächt ist, wobei diese Schwächungslinie ein Aufreißstück zur Freigabe des Produktwests einschließt und auf diesem Aufreißstück ein Abdeckteil partiell unlösbar aufgesiegelt ist, welches an seinem der Solltrennfuge abgewandten Ende mit dem oberen Packstoff und an seinem der Solltrennfuge zugewandten Ende mit dem Griffstück untrennbar verbunden ist, gelöst.

Um die wirkstoffhaltigen Produkte zu entnehmen, ist es somit erforderlich, die einzelne in der Sekundärverpackung befestigte Primärverpackung aufzureißen. Erst danach kann die Primärverpackung von der Sekundärverpackung getrennt werden. Die Kindersicherung kann nicht dadurch umgangen werden, dass die Primärverpackungen ungeöffnet aus der Sekundärverpackung entnommen werden und außerhalb der Sekundärverpackung aufbewahrt werden.

Weitere Einzelheiten der Erfindung ergeben sich aus den Unteransprüchen und der nachfolgenden Beschreibung schematisch dargestellter Ausführungsformen.
- Figur 1:: Verpackungseinheit;
- Figur 2:: Stapel der Beutel aus Figur 1;
- Figur 3:: Stapel verschlossener Primärverpackungen;
- Figur 4:: Stapel mit einer geöffneten Primärverpackung;
- Figur 5:: Stapel nach Entnahme eines wirkstoffhaltigen Produkts;
- Figur 6:: Stapel mit einer abgetrennten Primärverpackung;

- Figur 7:: Stapel aus Vierrandsiegelbeuteln;
- Figur 8:: Stapel aus Figur 7 mit einer geöffneten Primärverpackung;
- Figur 9:: Stapel aus Figur 7 nach der Entnahme eines wirkstoffhaltigen Produkts;
- Figur 10:: Stapel aus Figur 7 mit einer abgetrennten Primärverpackung;
- Figur 11:: Stapel aus Figur 2 mit gefalteten Griffstück;
- Figur 12:: Stapel aus Figur 7 mit Sicherung der Solltrennfuge;
- Figur 13:: Stapel aus Figur 7 mit einem Griffstück, das die Solltrennfuge und die Solltrennnaht sichert.

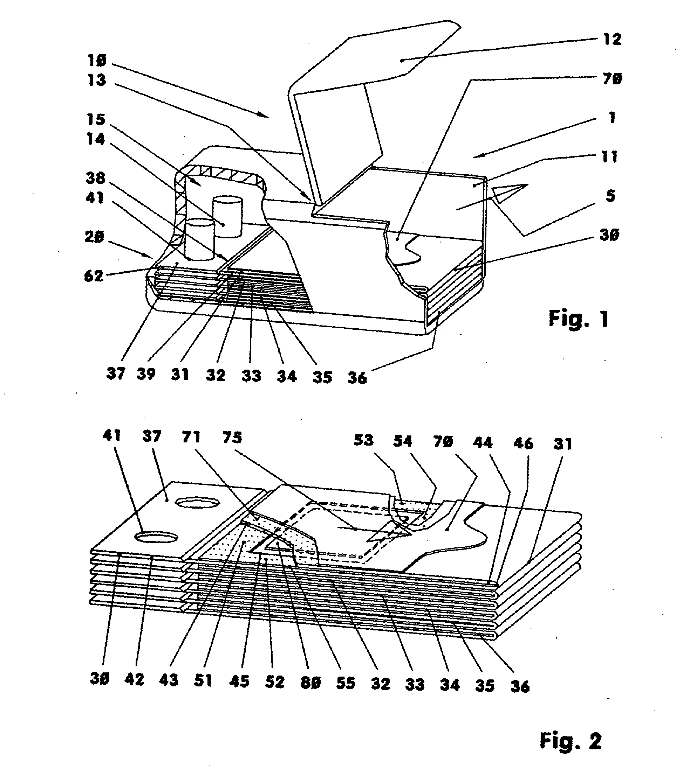

Die Figur 1 zeigt eine Verpackungseinheit (1). Dies ist eine Sammelpackung (1), bei der Primärverpackungen (30) wirkstoffhaltiger Produkte (80) in einer Sekundärverpackung (10) gestapelt angeordnet sind.

Die Sekundärverpackung (10) ist beispielsweise eine quaderförmige Schachtel. Sie besteht z.B. aus einem Gehäuse (11) und einem aufklappbaren Deckel (12). Der Deckel (12) ist in diesem Ausführungsbeispiel mittels eines Filmgelenks (13) mit dem Gehäuse (11) verbunden. Der Deckel (12) kann auch als Schiebedeckel, abnehmbarer Deckel, etc. ausgebildet sein. Der Verschluss dieser Sekundärverpackung (10) verfügt z.B. über eine hier nicht näher dargestellte Kindersicherung. Die Ecken und Kanten der Schachtel (10) sind beispielsweise abgerundet. Bei geschlossenem Deckel (12) ist die Schachtel (10) z.B. weitgehend dicht gegen das Eindringen von Fremdstoffen. Gegebenenfalls können am Deckel (12) und/oder am Gehäuse (11) zusätzliche Dichtelemente angeordnet sein, um zumindest während der Lagerung die Schachtel (10) hermetisch zu verschließen.

Im Innenraum (15) der Schachtel (10) ist ein Stapel (20) von Primärverpackungen (30) lösbar befestigt. Die Primärverpackungen (30) sind beispielsweise hermetisch und aromadicht versiegelte Siegelbeutel (30) mit je einem Griffstück (70). In der Schachtel (10) sind die Beutel (30) mittels zweier Bolzen (14) arretiert. Die Bolzen (14) durchdringen die Beutel (30) in zwei Durchgangsbohrungen (41), die hier einen großen Abstand zu den Außenrändern (62) der Beutel (30) haben. Statt der zwei dargestellten Bolzen (14) kann ein einzelner Bolzen, eine Klammer, etc. vorgesehen sein, die die Beutel (30) in der Schachtel (10) arretieren. Auch andere kraft- und/oder formschlüssige Befestigungen sind denkbar. In der in der Figur 1 dargestellten Sammelpackung (1) sind z.B. sechs Primärverpackungen (31 - 36) verpackt. Hierbei ist der Beutel (31) der oberste Beutel im Stapel (20), während der Beutel (36) der unterste der gestapelten Beutel (31 - 36) ist.

In jeder Primärverpackung (31 - 36) ist z.B. genau ein wirkstoffhaltiges Produkt (80) gelagert, vgl. Figur 2. Die wirkstoffhaltigen Produkte (80) sind beispielsweise dünne und flexible ein- oder mehrlagige Plättchen. Dies können beispielsweise transdermale Therapiesysteme, Wafer, Aromaplättchen, Indikatorstreifen oder -plättchen, etc. sein. Sie enthalten z.B. leicht flüchtige Wirkstoffe, die eine hermetisch dichte Primärverpackung (30) erfordern. Die wirkstoffhaltigen Produkte (80) liegen in Produktnestern (45) der Beutel (30). Diese Produktnester (45) sind größer als die in sie eingelegten Plättchen (80). In den einzelnen Beuteln (30) einer Sammelpackung (1) können - bei entsprechender Kennzeichnung - unterschiedliche wirkstoffhaltige Produkte (80) gelagert sein. Auch ist es denkbar, mehrere wirkstoffhaltige Produkte (80) in einem Beutel (30) anzuordnen.

Der einzelne Beutel (30), vgl. Figur 2, besteht aus mehreren Lagen miteinander verbundener Packstoffe (42, 43). Diese Packstoffe (42, 43) sind beispielsweise gasundurchlässige Kunststofffolien, die aus Packstoffbahnen hergestellt sind. Die Packstoffe (42, 43) sind beispielsweise aromadicht, wasserdicht und/oder sauerstoffdicht ausgeführt. Der einzelne Beutel (30) hat einen Befestigungsbereich (37), der die Bolzen (14) umgreift, und einen Aufnahmebereich (39), der das Produktnest (45) mit dem wirkstoffhaltigen Produkt (80) umfasst. Die beiden Bereiche (37, 39) sind in einer Solltrennfuge (38), z.B. einer Perforation, miteinander verbunden. Diese Perforation (38) liegt quer zur Entnahmerichtung (5) der Beutel (30) aus der Schachtel (10). Anstatt als Perforation (38) kann die Solltrennfuge (38) auch mittels einer Laserritzung, einer Lochung, einer Anritzung, etc. vorbereitet sein.

Die in den Figuren 1 und 2 dargestellten Siegelbeutel (30) bestehen jeweils aus einem unteren Packstoff (42), einem oberen Packstoff (43) und einem Griffstück (70). Der obere (43) und der untere Packstoff (42) sind Teil einer asymmetrisch gefalteten, rechteckigen Packstoffbahn (44). Hierbei ist der untere Packstoff (42) länger als der obere Packstoff (43). Der untere Packstoff (42) umfasst den Befestigungsbereich (37) und die Solltrennfuge (38). Die Falte (46), die den oberen (43) und den unteren Packstoff (42) unlösbar miteinander verbindet, liegt an der dem Befestigungsbereich (37) abgewandten Ende des Beutels (30).

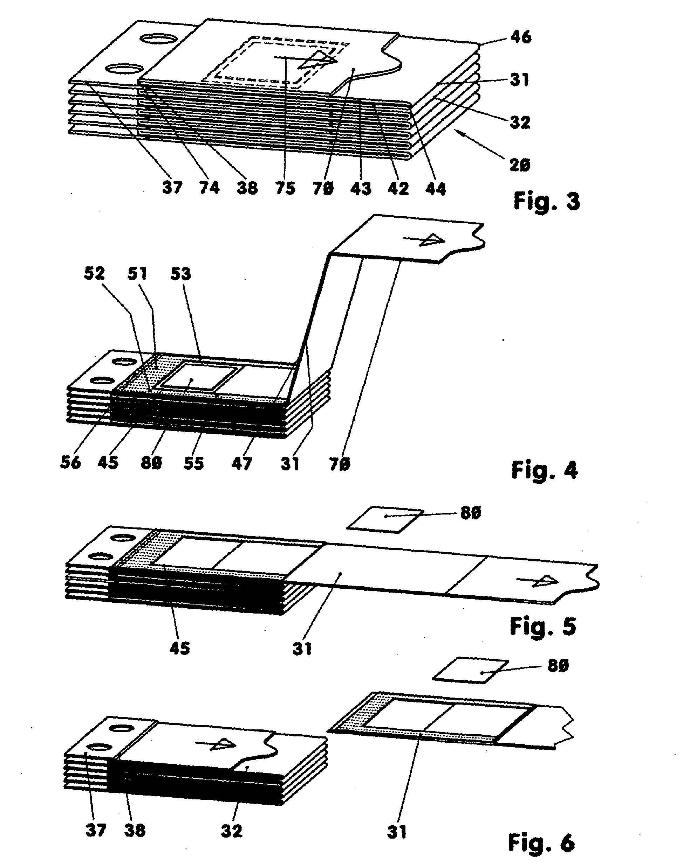

Der obere (43) und der untere Packstoff (42) sind beispielsweise in vier lösbaren Siegelnähten (51 - 54) miteinander versiegelt. Hierbei begrenzen zwei Längsnähte (52, 53) die beiden Längsseiten des Produktnests (45) und zwei Quernähte (51, 54) seine Querseiten. Statt als Vierrandsiegelbeutel kann dieser Beutel (30) auch als Dreirandsiegelbeutel ausgeführt sein, wobei dann die Quernaht (54) an der Falte (46) entfällt. Der Beutel (30) kann hierdurch kürzer ausgeführt werden. Die andere Quernaht (51) kann die Solltrennfuge (38) überdecken, so dass der obere Packstoff (43) mit dem unteren Packstoff (42) sowohl im Befestigungsbereich (37) als auch im Aufnahmebereich (39) verbunden ist, vgl. Figur 3. Die einzelnen gestapelten Beutel (31 - 36) können im Befestigungsbereich (37) miteinander verbunden sein.

Der Vierrandsiegelbeutel (30) kann auch derart ausgeführt sein, dass der obere (43) und der untere Packstoff (42) nicht mittels einer Falte (46) verbunden sind. Die Quernaht (51) und die Längsnähte (52, 53) sind dann als auftrennbare Nähte ausgeführt, während die Quernaht (54) eine untrennbare Naht ist.

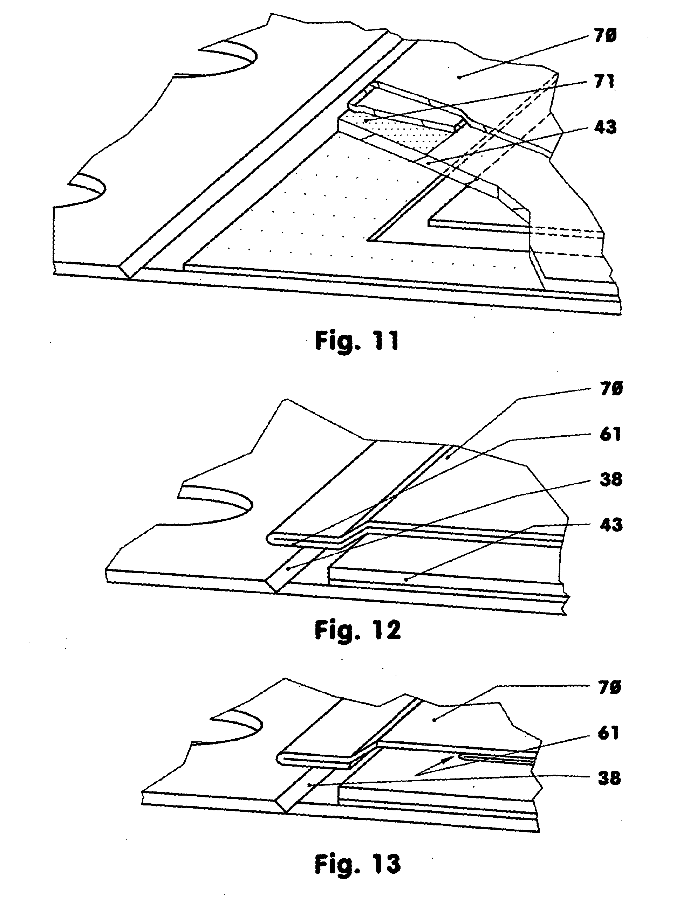

An den oberen Packstoff (43) ist in diesem Ausführungsbeispiel das Griffstück (70) unlösbar in einer Quernaht (71) angesiegelt. Das Griffstück (70) kann auch Teil der Packstoffbahn (44) sein und beispielsweise durch Umlegen des oberen Packstoffs (43) erzeugt werden. Auch ein Ansiegeln und Umlegen an den oberen Packstoff (43), vgl. Figur 11, ist denkbar. Das Griffstück (70) des Ausführungsbeispiels besteht beispielsweise aus einer Kunststofffolie, die halb so stark ist wie die Packstoffe (42, 43). Es kann aber auch aus dem gleichen Werkstoff wie die Packstoffe (42, 43) hergestellt sein. Die Länge des Griffstücks (70) beträgt beispielsweise zwei Drittel der Länge des oberen Packstoffs (43). Die Quernaht (71) oder die Falte zwischen dem oberen Packstoff (43) und dem Griffstück (70) kann die Solltrennfuge (38) überlappen.

Soll ein wirkstoffhaltiges Produkt (80) entnommen werden, wird zunächst der Deckel (12) geöffnet. Der Anwender sieht den Stapel (20) der Beutel (30), wobei vom obersten Beutel (31) das Griffstück (70) sichtbar ist. Der Bereich der Siegelnaht (51) ist vollständig in der Schachtel (10) verdeckt.

Die Entnahme eines wirkstoffhaltigen Produkts (80) wird nun anhand der Figuren 3 bis 6 beschrieben, in denen der Stapel (20) der Peelbeutel (31 - 36) ohne die Sekundärverpackung (10) dargestellt ist. Hierbei ist der Stapel (20) in der Figur 3 vergrößert gegenüber den Figuren 4 - 6 dargestellt. Die in diesen Figuren 3 bis 6 dargestellten Dreirandsiegelbeutel (31 - 36) sind jeweils aus einer einzigen Packstoffbahn (44) hergestellt. Der obere Packstoff (43) stößt in der Falte (46) an den unteren Packstoff (42). Das Griffstück (70) liegt zunächst, vgl. Figur 3, auf dem oberen Packstoff (43) auf und ist mit diesem mittels einer weiteren querliegenden Falte (74) unlösbar verbunden. Der obere Packstoff (43) überlappt die Solltrennfuge (38).

Der obere (43) und der untere Packstoff (42) sind mittels zweier Längsnähte (52, 53) und einer Quernaht (51), vgl. Figur 4, miteinander verbunden. Alle diese Nähte (51 - 53) sind auftrennbare Siegelnähte. Die Längsnähte (52, 53) sind an beiden Seiten des gegebenenfalls vertieften Produktnestes (45) angeordnet. Die Quernaht (51) liegt zu beiden Seiten der Solltrennfuge (38) und überdeckt sowohl Teile des Befestigungsbereichs (37) als auch Teile des Aufnahmebereichs (39). Gegebenenfalls kann auch ein Bereich des Griffstücks (70), der als schmaler Streifen an die Falte (74) angrenzt und parallel zu diesem ausgerichtet ist, lösbar an den oberen Packstoff (43) angesiegelt sein. Hiermit wird die Lage des Griffstücks (70) bei der Lagerung der Schachtel (10) fixiert. Zum Lösen dieser Naht sind nur sehr geringe Kräfte erforderlich.

Zum Öffnen des obersten Peelbeutels (31) zieht der Anwender das Griffstück (70) in der Richtung des z.B. eingravierten Pfeils (75). Je nach Ausbildung der Schachtel (10) kann er das Griffstück (70) mit einem steilen oder mit einem flachen Abziehwinkel ziehen. Hierbei wird zunächst die gegebenenfalls vorhandene Naht oder der Haftbereich zwischen dem Griffstück (70) und dem oberen Packstoff (43) aufgetrennt. Beim weiteren ziehen wird der obere Packstoff (43) entlang der Solltrennnaht (55) vom unteren Packstoff (42) abgepeelt, vgl. Figur 4. Die Solltrennnaht (55) umfasst in diesem Ausführungsbeispiel die auftrennbaren Siegelnähte (51 - 53). Sie begrenzt den Aufreißbereich (47) des Peelbeutels (31). Hierbei verhindert die Quernaht (51), die die Solltrennfuge (38) überlappt, ein Auftrennen der Perforation (38) bei geschlossenem Peelbeutel (31). Beim Abziehen des oberen Packstoffs (43) wird der Aufreißbereich (47) des Peelbeutels (30) geöffnet. Das wirkstoffhaltige Produkt (80) wird vollflächig freigelegt. Es kann nun ohne Schwierigkeiten mit den Fingern gegriffen werden und kann dann aus dem Produktnest (45) entnommen werden. Hierbei besteht keine Gefahr, dass das dünne und flexible Plättchen (80) beschädigt wird, selbst wenn es nur wenige Zehntelmillimeter stark ist.

Beim weiteren Ziehen des Griffstücks (70) wird der nun leere Peelbeutel (31) gestreckt, vgl. Figur 5. Der Aufreißbereich (47) ist nun vollständig geöffnet. Die auftrennbaren Siegelnähte (51 - 53) sind aufgetrennt.

Um die Schachtel (10) nach der Entnahme des wirkstoffhaltigen Produkts (80) zu schließen, muss zunächst der leere Beutel (31) entfernt werden. Hierzu wird z.B. mit einem Ruck am Griffstück (70) die Perforation (38) aufgetrennt, vgl. Figur 6. Da der durch die Quernaht (51) gebildete Sicherungsbereich (56) bereits aufgetrennt ist, ist die zum Auftrennen der Perforation (38) erforderliche Kraft beispielsweise ähnlich groß wie die Kraft zum Aufpeelen des Dreirandsiegelbeutels (31). Der leere, geöffnete Beutel (31) kann nun entsorgt werden. Der Anwender sieht in der Schachtel (10) den nächsten geschlossenen Beutel (32) mit seinem Griffstück (70).

Nun kann die - z.B. kindersichere - Schachtel (10) wieder geschlossen werden und an einem sicheren Ort aufbewahrt werden. Um z.B. die nächste Arzneimitteldosis zu entnehmen, kann der Anwender bei geöffneter Schachtel (10) mittels des nächsten Griffstücks (70) den nächsten Beutel (32) aufreißen.

Das wirkstoffhaltige Produkt (80) kann gegebenenfalls auch erst nach dem Abreißen des geöffneten Beutels (31) aus den Produktnest (45) entnommen werden. Das Produktnest (45) kann hierfür eine Sicherheitskante aufweisen, die ein Herausfallen des wirkstoffhaltigen Produkts (80) während des Abreißens des Beutels (31) verhindert.

Mit dieser Verpackungseinheit (1) ist eine sichere und beschädigungsfreie Entnahme des wirkstoffhaltigen Produkts (80) möglich. Nur geöffnete Beutel (30) können aus der Sekundärverpackung (10) entnommen werden. Hierdurch ist sichergestellt, dass keine Beutel (30) "auf Vorrat" entnommen werden und z.B. für Kinder zugänglich aufbewahrt werden.

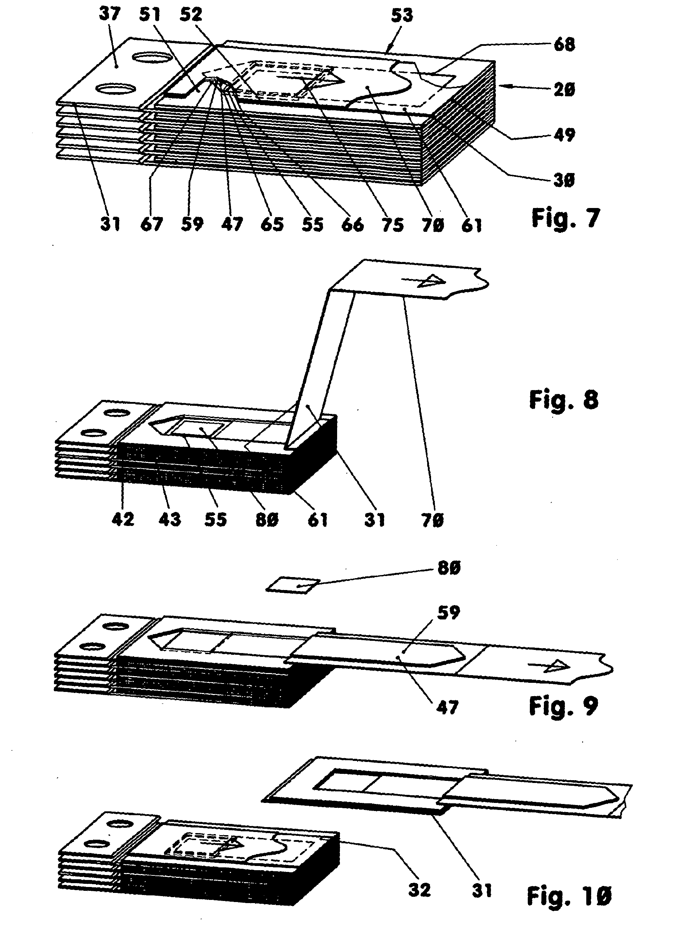

In den Figuren 7 - 10 ist als Primärverpackung (30) der wirkstoffhaltigen Produkte ein Vierrandsiegelbeutel (30) in der Ausführungsform eines Aufreißbeutels dargestellt. In der Figur 7 ist der Stapel (20) der Beutel (31 - 36) vergrößert gegenüber den Figuren 8 - 10 dargestellt. Auch jeder dieser Beutel (31 - 36) umfasst einen oberen (43) und einen unteren (42) Packstoff. Diese beiden Packstoffe (42, 43) sind mittels zweier Längsnähte (52, 53) und zweier Quernähte (51, 54), die das Produktnest (45) begrenzen, vgl. Figur 2, miteinander versiegelt. Diese Siegelnähte (51 - 54) sind nicht peelfähig und damit nicht auftrennbar. Der in den Figuren 7 - 10 dargestellte Siegelbeutel (30) kann auch als Dreirandsiegelbeutel (30) ausgeführt sein. Hierbei ist dann die obere (43) mit der unteren Packbahn (42) beispielsweise mittels einer Falte (46) unlösbar verbunden.

Der obere Packstoff (43) ist entlang einer Linie (65) mechanisch geschwächt, vgl. Figur 7. Diese Linie (65) hat drei Teilabschnitte (66 - 68). Zwei zueinander parallele Abschnitte (66, 68) fluchten beispielsweise mit den seitlichen Begrenzungen des Produktnests (45) und sind in der Richtung des Pfeils (75) auf dem Griffstück (70) bis an die Quernaht (54) geführt. Ein weiterer Abschnitt (67) dieser Linie (65) verbindet diese beiden Teillinien (66, 68). Er bildet eine in Richtung des Befestigungsbereichs (37) orientierte Spitze (67). Diese Spitze (67) liegt zwischen dem Produktnest (45) und dem Befestigungsbereich (37). Innerhalb des von der Linie (65) eingeschlossenen Bereichs sind der obere (43) und der untere Packstoff (42) nicht miteinander versiegelt. Die mechanische Schwächung ist beispielsweise eine mittels eines Lasers erzeugte Ritzung. Dabei werden die oberen Schichten des Packstoffs (43) abgetragen, ohne die unteren Schichten des Packstoffs (43), die für die Dichtigkeit des Beutels (30) verantwortlich sind, zu beschädigen. Der von der Linie (65) begrenzte Bereich des Packstoffes (43) wird im folgenden als Aufreißstück (59) bezeichnet.

Auf das Aufreißstück (59) ist ein Abdeckstück (61) partiell unlösbar aufgesiegelt. Das Abdeckstück (61) überlappt das Aufreißstück (59) und steht beispielsweise auf beiden Längsseiten um die Hälfte der Breite einer Siegelnaht (52, 53) über. Seine Länge entspricht beispielsweise der Länge des oberen Packstoffs (43). An seinem in Richtung des Befestigungsbereichs (37) orientierten Endes ist auf das Abdeckstück (61) das Griffstück (70) beispielsweise mit einer untrennbaren Siegelnaht (71), vgl. Figur 11, aufgesiegelt. Das Abdeckstück (61) kann auch auf der dem Befestigungsbereich (37) zugewandten Seite gefaltet sein, um das Griffstück (70) zu bilden. Auch in diesem Ausführungsbeispiel trägt das Griffstück (70) eine Markierung (75), um die Entnahmerichtung (5) der Beutel (30) zu kennzeichnen. Das Griffstück (70) kann zusätzlich mittels einer Naht oder mittels eines Haftbereichs mit dem Abdeckstück (61) verbunden sein.

Das Öffnen des ersten Beutels (31) erfolgt analog dem Öffnen des in den Figuren 3 - 6 dargestellten Beutels (31). Das Griffstück (70) wird mit den Fingern in Richtung des Markierungspfeils (75) gezogen. Hierbei zieht das Griffstück (70) das Abdeckstück (61) mit. Das Abdeckstück (61) wiederum reißt das Aufreißstück (59), beginnend von der Spitze (67), entlang der durch die Linie (65) markierten Solltrennnaht (55) aus dem oberen Packstoff (43). Der nun geöffnete Aufreißbereich (47) legt das wirkstoffhaltige Produkt (80) im Produktnest (45) vollflächig frei, vgl. Figur 8. Das wirkstoffhaltige Produkt (80) kann nun ohne Beschädigungen entnommen werden. Auch bei dieser Ausführungsform kann die Solltrennfuge (38) zusätzlich geschützt sein, beispielsweise durch ein auftrennbares Versiegeln des Abdeckstücks (61) auf dem Befestigungsbereich (37), vgl. Figur 12.

Beim weiteren Ziehen des Griffstücks (70) wird das Aufreißstück (59) bis zum Ende der Linie (65) aufgerissen. Die Quernaht (54) verhindert ein weiteres Aufreißen. Hiernach wird die Zugkraft auf die Perforation (38) übertragen. Der Beutel (31), der nun z.B. leer ist, wird beispielsweise mittels eines kurzen, kräftigen Rucks entlang der Perforation (38) abgerissen. Nach der Entnahme des ersten geöffneten Beutels (31) verbleiben in der Schachtel (10) nur verschlossene Beutel (32 - 36), die jeweils ein wirkstoffhaltiges Produkt (80) enthalten. Diese Beutel (32 - 36) können in der gleichen Vorgehensweise entnommen werden.

Somit ist sichergestellt, dass sich keine ungeöffneten Beutel (30) außerhalb der Schachtel (10) befinden.

Das Griffstück (70) kann auch so ausgebildet sein, dass es sowohl die Solltrennfuge (38) als auch das Abdeckstück (61) überdeckt, vgl. Figur 13. Beim Ziehen des Griffstücks (70) wird dann die Solltrennnaht (55) geöffnet und die Solltrennfuge (38) freigelegt.

### Bezugszeichenliste:

- 1: Verpackungseinheit, Sammelpackung
- 5: Entnahmerichtung

- 10: Sekundärverpackung, Behälter, Schachtel
- 11: Gehäuse
- 12: Deckel
- 13: Filmgelenk
- 14: Bolzen
- 15: Innenraum

- 20: Stapel

- 30: Primärverpackung, Siegelbeutel
- 31 - 36: Primärverpackungen, Siegelbeutel
- 37: Befestigungsbereich
- 38: Solltrennfuge, Perforation
- 39: Aufnahmebereich

- 41: Durchgangsbohrungen
- 42: unterer Packstoff
- 43: oberer Packstoff
- 44: Packstoffbahn
- 45: Produktnest
- 46: Falte
- 47: Aufreißbereich

- 49: Kante von (43)

- 51: Siegelnaht, Quernaht
- 52: Siegelnaht, Längsnaht
- 53: Siegelnaht, Längsnaht
- 54: Siegelnaht, Quernaht

- 55: Solltrennnaht
- 56: Sicherungsbereich

- 59: Aufreißstück

- 61: Abdeckstück
- 62: Außenränder

- 65: Linie
- 66: Teilabschnitt von (65)
- 67: Teilabschnitt von (65), Spitze
- 68: Teilabschnitt von (65)

- 70: Griffstück
- 71: Quernaht

- 74: Falte
- 75: Markierung, Pfeil

- 80: wirkstoffhaltiges Produkt, Plättchen

## Patentansprüche

1. Verpackungseinheit (1), die mindestens ein wirkstoffhaltiges Produkt (80), mindestens eine Primärverpackung (30, 31, 32, 33, 34, 35, 36) und eine Sekundärverpackung (10) umfasst, wobei zumindest ein wirkstoffhaltiges Produkt (80) in jeder geschlossenen Primärverpackung (30, 31, 32, 33, 34, 35, 36) versiegelt gelagert ist und wobei die Primärverpackungen (30, 31, 32, 33, 34, 35, 36) in der Sekundärverpackung (10) gestapelt angeordnet und mittels Solltrennfugen (38) zumindest bereichsweise lösbar befestigt sind, wobei
- jede Primärverpackung (30) ein Siegelbeutel (30) mit einem Griffstück (70) ist,
- der einzelne Siegelbeutel (30) eine Solltrennnaht (55) hat, die einen Aufreißbereich (47) begrenzt,
- das Griffstück (70) mit dem Aufreißbereich (47) verbunden ist,
- der mittels eines Ziehens des Griffstücks (70) geöffnete Aufreißbereich (47) das wirkstoffhaltige Produkt (80) freilegt und
- ein weiteres Ziehen des Griffstücks (70) die Solltrennfuge (38) trennt,
- der Siegelbeutel (30) einen oberen (43) und einen unteren Packstoff (42) aufweist, die zumindest an der der Solltrennfuge (38) abgewandten Seite des Siegelbeutels (30) untrennbar miteinander verbunden sind,
**dadurch gekennzeichnet,**
**dass** der untere und obere Packstoff (42, 43) an der der Solltrennfuge (38) abgewandten Seite des Beutels mittels einer querliegenden Falte (46) unlösbar miteinander verbunden sind, wobei die Solltrennnähte (55) des Siegelbeutels eine auftrennbare gesiegelte Quernaht (51) an der der Solltrennfuge (38) zugewandten Seite des Beutels und zwei in Zugrichtung des Griffstücks (70) orientierte auftrennbar gesiegelte Längsnähte (52, 53) sind, die das Produktnest (45) begrenzen, und wobei das Griffstück (70) auf dem oberen Packstoff (43) aufliegt und mit diesem an der der Solltrennfuge (38) zugewandten Seite des Beutels mittels einer querliegenden Falte (74) unlösbar verbunden ist.

2. Verpackungseinheit (1), die mindestens ein wirkstoffhaltiges Produkt (80), mindestens eine Primärverpackung (30, 31, 32, 33, 34, 35, 36) und eine Sekundärverpackung (10) umfasst, wobei zumindest ein wirkstoffhaltiges Produkt (80) in jeder geschlossenen Primärverpackung (30, 31, 32, 33, 34, 35, 36) versiegelt gelagert ist und wobei die Primärverpackungen (30, 31, 32, 33, 34, 35, 36) in der Sekundärverpackung (10) gestapelt angeordnet und mittels Solltrennfugen (38) zumindest bereichsweise lösbar befestigt sind, wobei
- jede Primärverpackung (30) ein Siegelbeutel (30) mit einem Griffstück (70) ist,
- der einzelne Siegelbeutel (30) eine Solltrennnaht (55) hat, die einen Aufreißbereich (47) begrenzt,
- das Griffstück (70) mit dem Aufreißbereich (47) verbunden ist,
- der mittels eines Ziehens des Griffstücks (70) geöffnete Aufreißbereich (47) das wirkstoffhaltige Produkt (80) freilegt und
- ein weiteres Ziehen des Griffstücks (70) die Solltrennfuge (38) trennt,
- der Siegelbeutel (30) einen unteren und einen oberen Packstoff (42, 43) aufweist, die mittels untrennbarer Siegelnähte (51, 52, 53, 54) miteinander verbunden sind, welche das Produktnest (45) begrenzen,
**dadurch gekennzeichnet,**
**dass** der obere Packstoff (43) entlang einer Linie (65) mechanisch geschwächt ist, wobei diese Schwächungslinie ein Aufreißstück (59) zur Freigabe des produktwests (45) einschließt und auf diesem Aufreißstück (59) ein Abdeckteil (61) partiell unlösbar aufgesiegelt ist, welches an seinem der Solltrennfuge (38) abgewandten Ende mit dem oberen Packstoff (43) und an seinem der Solltrennfuge (38) zugewandten Ende mit dem Griffstück (70) untrennbar verbunden ist.

3. Verpackungseinheit nach Anspruch 1 oder 2,
**dadurch gekennzeichnet,**
**dass** die Solltrennfuge (38) eine Perforation (38) ist.

4. Verpackungseinheit nach Anspruch 1 oder 2,
**dadurch gekennzeichnet,**
**dass** die Siegelbeutel (30) form- und/oder kraftschlüssig in der Sekundärverpackung (10) befestigt sind.

## Claims

1. Packaging unit (1), which comprises at least one active-substance-containing product (80), at least one primary packaging (30, 31, 32, 33, 34, 35, 36) and a secondary packaging (10), wherein at least one active-substance-containing product (80) is stored sealed in each closed primary packaging (30, 31, 32, 33, 34, 35, 36), and wherein the primary packagings (30, 31, 32, 33, 34, 35, 36) are arranged stacked in the secondary packaging (10) and are secured releasably, at least in some areas, by predetermined partition lines (38), wherein
- each primary packaging (30) is a sealed pouch (30) with a gripping piece (70),
- the individual sealed pouch (30) has a predetermined partition seam (55) that delimits a tear-open area (47),
- the gripping piece (70) is connected to the tear-open area (47),
- the tear-open area (47), opened by pulling the gripping piece (70), exposes the active-substance-containing product (80), and
- further pulling of the gripping piece (70) separates the predetermined partition line (38),
- the sealed pouch (30) has an upper packing material (43) and a lower packing material (42), which are connected inseparably to each other at least on the side of the sealed pouch (30) directed away from the predetermined partition line (38), **characterized in that** the lower and upper packing materials (42, 43), on the side of the pouch directed away from the predetermined partition line (38), are connected non-releasably to each other by means of a transverse fold (46), wherein the predetermined partition seams (55) of the sealed pouch are one separable sealed transverse seam (51), on the side of the pouch directed toward the predetermined partition line (38), and two sealed and separable longitudinal seams (52, 53) which are oriented in the direction of pulling of the gripping piece (70), said seams delimiting the product nest (45), and wherein the gripping piece (70) lies on the upper packing material (43) and, on the side of the pouch directed toward the predetermined partition line (38), is connected non-releasably to the upper packing material (43) by means of a transverse fold (74).

2. Packaging unit (1), which comprises at least one active-substance-containing product (80), at least one primary packaging (30, 31, 32, 33, 34, 35, 36) and a secondary packaging (10), wherein at least one active-substance-containing product (80) is stored sealed in each closed primary packaging (30, 31, 32, 33, 34, 35, 36), and wherein the primary packagings (30, 31, 32, 33, 34, 35, 36) are arranged stacked in the secondary packaging (10) and are secured releasably, at least in some areas, by predetermined partition lines (38), wherein
- each primary packaging (30) is a sealed pouch (30) with a gripping piece (70),
- the individual sealed pouch (30) has a predetermined partition seam (55) that delimits a tear-open area (47),
- the gripping piece (70) is connected to the tear-open area (47),
- the tear-open area (47), opened by pulling the gripping piece (70), exposes the active-substance-containing product (80), and
- further pulling of the gripping piece (70) separates the predetermined partition line (38),
- the sealed pouch (30) has a lower packing material (42) and an upper packing material (43), which are connected to each other by means of inseparable sealed seams (51, 52, 53, 54), which delimit the product nest (45),
**characterized in that** the upper packing material (43) is mechanically weakened along a line (65), wherein this weakening line includes a tear-open piece (59) for releasing the product nest (45), and a cover piece (61) is partially sealed non-releasably on this tear-open piece (59), which cover piece (61), at its end directed away from the predetermined partition line (38), is connected inseparably to the upper packing material (43) and, at its end directed toward the predetermined partition line (38), is connected inseparably to the gripping piece (70).

3. Packaging unit according to Claim 1 or 2, **characterized in that** the predetermined partition line (38) is a perforation (38).

4. Packaging unit according to Claim 1 or 2, **characterized in that** the sealed pouches (30) are secured with a form fit and/or force fit in the secondary packaging (10).

## Revendications

1. Unité d'emballage (1), qui comprend au moins un produit (80) comprenant une substance active, au moins un emballage primaire (30, 31, 32, 33, 34, 35, 36) et un emballage secondaire (10), au moins un produit (80) comprenant une substance active étant stocké sous forme scellée dans chaque emballage primaire fermé (30, 31, 32, 33, 34, 35, 36) et les emballages primaires (30, 31, 32, 33, 34, 35, 36) étant disposés en pile dans l'emballage secondaire (10) et étant fixés de manière amovible au moins en partie au moyen de joints à zone de séparation (38),
- chaque emballage primaire (30) étant un sachet scellable (30) avec une partie de préhension (70),
- le sachet scellable individuel (30) ayant un cordon de zone de séparation (55) qui limite une zone de déchirure (47),
- la partie de préhension (70) étant connectée à la zone de déchirure (47),
- la zone de déchirure (47) ouverte en tirant sur la partie de préhension (70) exposant le produit (80) comprenant une substance active et
- une traction supplémentaire de la partie de préhension (70) séparant le joint à zone de séparation (38),
- le sachet scellable (30) présentant une matière d'emballage supérieure (43) et inférieure (42), qui sont connectées l'une à l'autre de manière inséparable au moins au niveau du côté du sachet scellable (30) opposé au joint à zone de séparation (38),
**caractérisée en ce que**
la matière d'emballage inférieure et supérieure (42, 43) sont connectées l'une à l'autre de manière non détachable au niveau du côté du sachet opposé au joint à zone de séparation (38) au moyen d'un pli transversal (46), les cordons de zone de séparation (55) du sachet scellable étant un cordon transversal scellé ouvrable (51) du côté du sachet tourné vers le joint à zone de séparation (38) et deux cordons longitudinaux (52, 53) scellés de manière ouvrable orientés dans la direction de traction de la partie de préhension (70), qui limitent la cavité du produit (45), et la partie de préhension (70) reposant sur la matière d'emballage supérieure (43) et étant connectée de manière non détachable à celle-ci au niveau du côté du sachet tourné vers le joint à zone de séparation (38) au moyen d'un pli transversal (74).

2. Unité d'emballage (1), qui comprend au moins un produit (80) comprenant une substance active, au moins un emballage primaire (30, 31, 32, 33, 34, 35, 36) et un emballage secondaire (10), au moins un produit (80) comprenant une substance active étant stocké sous forme scellée dans chaque emballage primaire fermé (30, 31, 32, 33, 34, 35, 36) et les emballages primaires (30, 31, 32, 33, 34, 35, 36) étant disposés en pile dans l'emballage secondaire (10) et étant fixés de manière amovible au moins en partie au moyen de joints à zone de séparation (38),
- chaque emballage primaire (30) étant un sachet scellable (30) avec une partie de préhension (70),
- le sachet scellable individuel (30) ayant un cordon de zone de séparation (55) qui limite une zone de déchirure (47),
- la partie de préhension (70) étant connectée à la zone de déchirure (47),
- la zone de déchirure (47) ouverte en tirant sur la partie de préhension (70) exposant le produit (80) comprenant une substance active et
- une traction supplémentaire de la partie de préhension (70) séparant le joint à zone de séparation (38),
- le sachet scellable (30) présentant une matière d'emballage supérieure (43) et inférieure (42), qui sont connectées l'une à l'autre au moyen de cordons scellés non séparables (51, 52, 53, 54), qui limitent la cavité du produit (45),
**caractérisée en ce que**
la matière d'emballage supérieure (43) est affaiblie mécaniquement le long d'une ligne (65), cette ligne d'affaiblissement renfermant une partie d'ouverture déchirable (59) pour ouvrir la cavité du produit (45), et une partie de recouvrement (61) étant scellée de manière partiellement non détachable sur cette partie d'ouverture déchirable (59), laquelle partie de recouvrement est connectée de manière non séparable à son extrémité opposée au joint à zone de séparation (38), à la matière d'emballage supérieure (43) et à son extrémité tournée vers le joint à zone de séparation (38), à la partie de préhension (70).

3. Unité d'emballage selon la revendication 1 ou 2,
**caractérisée en ce que**
le joint à zone de séparation (38) est une perforation (38).

4. Unité d'emballage selon la revendication 1 ou 2,
**caractérisée en ce que**
les sachets scellables (30) sont fixés par engagement par correspondance géométrique et/ou par force dans l'emballage secondaire (10).
